# EUROPEAN PATENT SPECIFICATION

(11) **EP 3 069 130 B1**
(45) Date of publication and mention of the grant of the patent: **31.01.2018**
(21) Application number: 14796148.6
(22) Date of filing: 12.11.2014
(51) Int. Cl.: G01N 27/12

(54) **INTEGRATED GAS SENSOR AND RELATED MANUFACTURING PROCESS**
INTEGRIERTER GASSENSOR UND ZUGEHÖRIGES HERSTELLUNGSVERFAHREN
CAPTEUR DE GAZ INTÉGRÉ ET SON PROCÉDÉ DE FABRICATION

(30) Priority: 12.11.2013 US 201361903073 P
(43) Date of publication of application: 21.09.2016
(73) Proprietor: LFoundry S.r.l., 67051 Avezzano (IT)
(72) Inventor: MONTANYA SILVESTRE, Josep, E-08193 Bellaterra (ES)
(74) Representative: Nannucci, Lorenzo
(86) International application number: PCT/EP2014/074420
(87) International publication number: WO 2015/071337

(56) References cited:
- EP-A1- 2 554 980
- EP-A1- 2 645 091
- US-A- 4 197 089
- US-A1- 2006 185 980
- US-A1- 2006 267 051

## Description

### TECHNICAL FIELD

The present invention relates to an integrated gas sensor and to a related manufacturing process.

### BACKGROUND ART

As is known, MEMS (Micro-ElectroMechanical System) sensors are commonly employed in electronic devices, in particular in portable or mobile electronic devices, such as smartphones, tablets or similar, or wearable equipments, such as smart watches or similar.

It is underlined that the term "MEMS" is herein intended to encompass also nano-structures, or in general structures with sub-micrometric dimensions.

MEMS sensors include e.g. motion sensors, such as accelerometers or gyroscopes, force sensors or environmental sensors, such as pressure sensors, humidity or gas sensors.

In particular, gas sensors are known, using a metal oxide as a sensing, or detection, element. The metal oxide changes its electrical conductivity, and hence its electrical resistance, due to the presence of a certain gas (or a number of gases), when it is heated at a given temperature (or temperatures).

MEMS sensors are generally manufactured with standard techniques of the semiconductor industry, starting from semiconductor substrates or wafers, e.g. made of silicon. The resulting MEMS sensors are usually integrated in a die and then housed in a package.

Integrated electronic circuits, so called ASICs (Application Specific Integrated Circuits) are operatively coupled to MEMS sensors, and usually integrated in a separated die of semiconductor material, which may be conveniently packaged with the MEMS sensor die within a single package, thus providing a compact packaged device.

In designing MEMS sensors and the related integrated electronic circuits, particularly for portable electronics applications, there is a continuing strive to reduce the size of the resulting devices, the power consumption and the overall manufacturing costs, at a same time guaranteeing desired electrical and mechanical performances.

EP 2 554 980 A1 discloses an integrated sensor, wherein a gas sensing element is formed on a passivation and metallization stack; a heating element, distinct from the gas sensing element, is formed within the stack.

US 2006/267051 A1 discloses a metal oxide sensor provided on a semiconductor substrate, and a related manufacturing process.

EP 2 645 091 A1 discloses an integrated circuit comprising a gas sensor and a related manufacturing process. The integrated circuit includes a semiconductor substrate having a major surface, and a thermal conductivity based gas sensor having an electrically resistive sensor element located on the major surface, for exposure to a gas to be sensed.

US 4 197 089 A discloses a sensor for the quantitative determination of reduction of a gas, including a thin tungsten oxide film detector element, coupled to a heater pad.

US 2006/185980 A1 discloses a ceramic gas sensor integrated with a semiconductor circuit, and a related manufacturing process.

The present Applicant has realized that known integrated gas sensors and related manufacturing processes are not fully satisfactory in terms of the above requirements, e.g. in terms of costs, size and power consumption.

### DISCLOSURE OF INVENTION

The aim of the present invention is consequently to provide an improved integrated gas sensor and manufacturing process.

According to the present invention, an integrated gas sensor and a related manufacturing process are consequently provided, as defined in the annexed claims.

### BRIEF DESCRIPTION OF THE DRAWINGS

For a better understanding of the present invention, preferred embodiments thereof are now described purely by way of non-limiting example and with reference to the attached drawings, wherein:
- Figure 1 shows a schematic cross section of an integrated gas sensor, according to an embodiment of the present solution;
- Figures 2a-2g are schematic cross sections of the integrated gas sensor of Figure 1, in subsequent steps of a related manufacturing process;
- Figure 3 is an equivalent circuit diagram of a gas sensing element in the integrated gas sensor of Figure 1;
- Figure 4 is a plot of electrical quantities related to the integrated gas sensor;
- Figure 5 is a schematic block diagram of the integrated gas sensor, in a possible embodiment of the present solution;
- Figure 6 is a flow chart of operations performed by a control unit in the integrated gas sensor, according to an embodiment of the present solution;
- Figures 7 and 8 are schematic plan views of a sensing element in the integrated gas sensor, according to further embodiments of the present solution; and
- Figure 9 is a schematic depiction of a mobile device embedding the integrated gas sensor, according to a further aspect of the present solution.

### BEST MODE FOR CARRYING OUT THE INVENTION

As will be discussed in detail in the following, an embodiment of the present solution envisages manufacturing of an integrated gas sensor using mostly standard CMOS processing steps and materials, and in particular providing the integrated gas sensor within the interconnection layers (stacked metal and dielectric layers) of a typical CMOS integrated structure. In particular, as detailed in the following, one aspect of the present solution envisages use of a standard CMOS process and an additional etching post-process, in particular a vHF etching.

Moreover, a further aspect of the present solution envisages integration of a metal element, in particular a tungsten filament or wire, which is designed to be oxidized in order to allow measure of the concentration of one or more gases to be detected; the tungsten wire may have a very thin width or thickness, lower than 1 µm (being defined in this case a "tungsten nanowire").

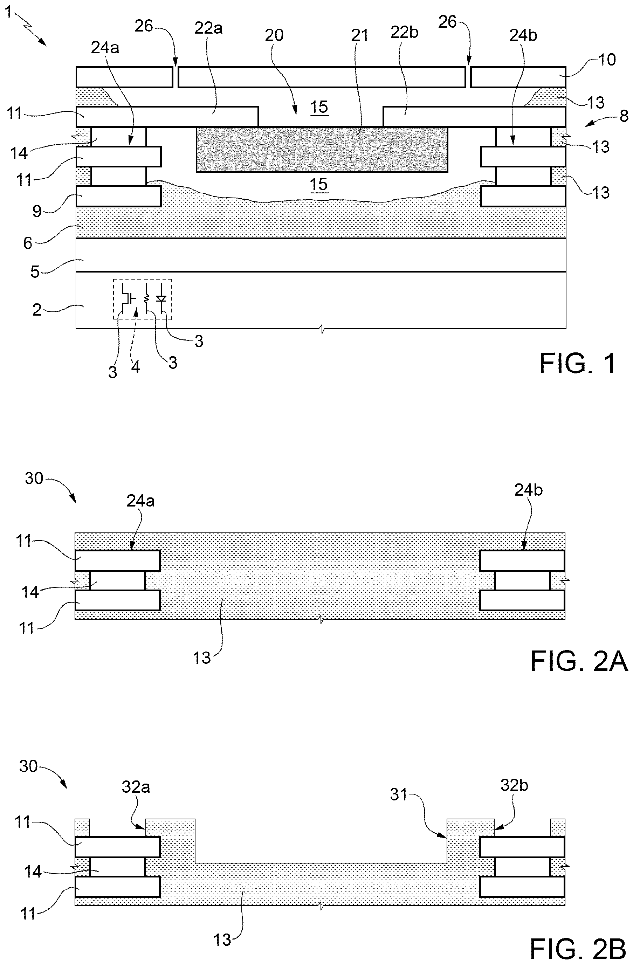

As shown in Figure 1, an integrated gas sensor 1, according to one embodiment of the present solution, includes a substrate 2 of semiconductor material, e.g. a silicon substrate.

In a known manner, which is not discussed in detail herein, electric/electronic components, shown schematically and denoted with 3, may be provided within the substrate 2, e.g. transistors, resistors, diodes, electrical connections and similar.

In particular, the above electric/electronic components 3 are designed to provide an integrated electronic circuit (ASIC) 4 of the integrated gas sensor 1; ASIC 4 is designed to be operatively coupled to a gas sensing structure of the integrated gas sensor 1, and to implement processing operations, e.g. amplification and filtering operations, on detected electrical signals.

The integrated gas sensor 1 includes, arranged above the substrate 2, a bottom dielectric layer, usually denoted as Inter Level Dielectric (ILD) layer, which may include a bottom region 5 of doped oxide (for example, BPSG or phosphosilicate glass (PSG)) and a top region 6 of non-doped oxide.

The integrated gas sensor 1 moreover includes, above the bottom dielectric layer 5, 6, a structure of interconnection layers 8, including a number of stacked layers of conductor material, e.g. of a metal such as aluminium, and dielectric layers, e.g. of silicon oxide.

In particular, the structure of interconnection layers 8 includes a bottom conductive layer 9 and a top conductive layer 10, which is designed to provide an external contact interface allowing contact from the outside towards the bottom layers and the ASIC 4 integrated within the substrate 2.

Between the bottom conductive layer 9 and the top conductive layer 10, the structure of interconnection layers 8 includes a number (equal to two in the non limitative example of Figure 1) of additional conductive layers 11, e.g. of a metal such as aluminium.

The conductive layers 9, 10, 11 are separated from one another by dielectric material layers 13, e.g. of silicon oxide, and conductive vias 14 traverse the dielectric material layers 13 in order to provide electrical conductive paths to interconnect the conductive layers 9, 10, 11, according to desired electrical connection paths. Typically, conductive vias 14 include tungsten for a Al back-end CMOS process.

Moreover, according to an aspect of the present solution, a hollow space 15 is defined within the structure of interconnection layers 8, via selective removal of portions of one or more of the dielectric layers 13 and definition of the conductive layers 11.

The integrated gas sensor 1 further includes a gas sensing structure 20, which comprises a sensing element 21, and first and second electrodes 22a, 22b, electrically connected to a respective end portion of the sensing element 21.

In particular, the sensing element 21 includes at least one metal oxide element, for example in the form of a tungsten oxide filament or wire (which may be linear or have a meander or serpentine configuration in a top plan view); the sensing element 21 may include a tungsten oxide nanowire (e.g. WO₃).

As an alternative, the sensing element 21 may include an array of elements, for example an array of tungsten wires (and, possibly, a corresponding number of electrodes).

In the example shown, both first and second electrodes 22a, 22b are formed in a same conductive layer 11 (in particular, the conductive layer 11 adjacent to the top conductive layer 10); moreover, in the same example, the sensing element 21 is suspended within the hollow space 15, attached to the first and second electrodes 22a, 22b of the conductive layer 11.

It is however underlined that other options are possible with respect to the arrangement of the sensing element 21. For instance, the tungsten element could be buried into one or more of the dielectric layer 13, instead of being hanged on a conductive layer 11, still being electrically connected thereto via the first and second electrodes 22a, 22b.

The integrated gas sensor 1 further includes conductive columns 24a, 24b, formed by a stack-up of portions of the conductive layers 11 and of conductive vias 14 defined through respective dielectric material layers 13; the conductive columns 24a, 24b provide electrical connections from the first and second electrodes 22a, 22b to the bottom conductive layer 9.

Advantageously, the sensing element 21 may be formed in the same layer and with the same processing steps of one or more of the conductive vias 14, which in this case are also of metal material, e.g. tungsten. In other words, the sensing element 21 is formed, at least in part, with a standard step of the CMOS processing, i.e. the formation of vias 14 in the structure of interconnection layers 8.

In a manner not shown in detail, further electrical connections are provided through the bottom dielectric layer 5, 6, in order to define electrical connections toward the ASIC 4 integrated within the substrate 2, and in order to carry electrical signals detected by the gas sensing structure 20 towards the same ASIC 4.

In a manner not shown in detail, further electrical connections are also provided by means of the conductive layers 9, 10, 11, in order to carry electrical signals (e.g. being a function of the processing operations performed by the ASIC 4 on the detected electrical signals) towards the top conductive layer 11 and the outside of the integrated gas sensor 1.

The top conductive layer 11 has a number of through holes 26, through which (as discussed in detail below) an etching agent, such as HF (hydrofluoric acid) may be introduced towards the structure of interconnection layers 8, in particular for the formation of the hollow space 15.

A passivation layer (here not shown) may be provided on the top conductive layer 11, properly configured to allow access to the underlying hollow space 15 from the outside, so as to allow environment gas sensing by the sensing element 21.

Tungsten oxides in general, and WO₃ in particular, change their electrical resistivity depending on the presence of certain gas molecules, like NOx, COx, ethanol, water, ozone, among others. This change of electrical resistivity is also dependent on the temperature of the tungsten element.

According to an aspect of the present solution, in order to sense the gas concentration, a time sweep of a current or voltage applied to the sensing element 21, via the first and second electrodes 22a, 22b, is performed, so as to generate a corresponding sweep of its temperature. The resistance of the sensing element 21 is measured during the sweep, for example again via the first and second electrodes 22a, 22b.

Heating of the sensing element 21 is thus performed via the Joule effect, without additional heaters being required; in other words, "self-heating" of the sensing element 21 is implemented.

Application of the current and measuring of the resistance may be advantageously performed via the ASIC 4 integrated within the substrate 2; as an alternative, external electronic equipment may be used, possibly interfacing with the first and second electrodes 22a, 22b and/or the ASIC 4.

At each temperature, the sensitivity of the sensing element 21 to each gas is different; accordingly, it is possible to determine the composition of a set of gases in the air and measure the concentrations thereof.

Alternatively, if the sensing element 21 includes an array of oxide tungsten wires, a different current or voltage may be applied to each one of them (so as to reach a temperature suitable for sensing of a respective gas), in order to measure the concentration of each gas at once, without having to sweep the current through a period of time.

Still another option is to have a sensing element 21 including an array of oxide tungsten wires, each having a different size and/or shape, and connected in series or parallel; in this case, due to the different size and/or shape, each tungsten wire is set at a different temperature, when a same current or voltage is applied. In all the cases envisaging an array, means to measure the voltage or current at each individual oxide tungsten wire are provided, in order to sense the resistance of each element due to the applied current or voltage.

A manufacturing process of the integrated gas sensor 1 is now discussed, with initial reference to Figure 2a.

The process starts with an almost finished CMOS wafer or die, i.e. after the electric/electronic components 3 have been formed within the substrate 2, in order to provide the ASIC 4, and after the bottom dielectric layer 5, 6 and at least part of the structure of interconnection layers 8 has been formed.

By way of example, Figure 2a shows a starting CMOS wafer 30 (of which only the portion relevant to the present discussion is shown, for clarity reasons), where conductive layers 11 have already been defined (according to standard CMOS processing techniques), in order to define a bottom portion of the conductive columns 24a, 24b, above the bottom conductive layer 9 (here not shown). In particular, the conductive columns 24a, 24b have been previously formed by definition of the corresponding conductive layers 11, etching of the corresponding dielectric layers 13 and formation of the conductive vias 14.

An upper portion of the dielectric layers 13, e.g. including silicon oxide (SiO₂), is then etched, figure 2b, and patterned, using a suitable mask and standard CMOS step, in order to define a central cavity 31, in the region between the conductive columns 24a, 24b, and lateral recesses 32a, 32b, exposing a central portion of the underlying conductive layer 11.

Etching stops at the conductive layer 11, while central cavity 31 extends through the dielectric material for a greater depth with respect to the lateral recesses 32a, 32b. The bottom wall of central cavity 31 thus extends below the level of the uppermost conductive layer 11, into the underlying dielectric layer 13.

Moreover, the shape of the central cavity 31 (in top plan view) is a function of the desired conformation of the sensing element 21, which will be later formed within the same central cavity 31.

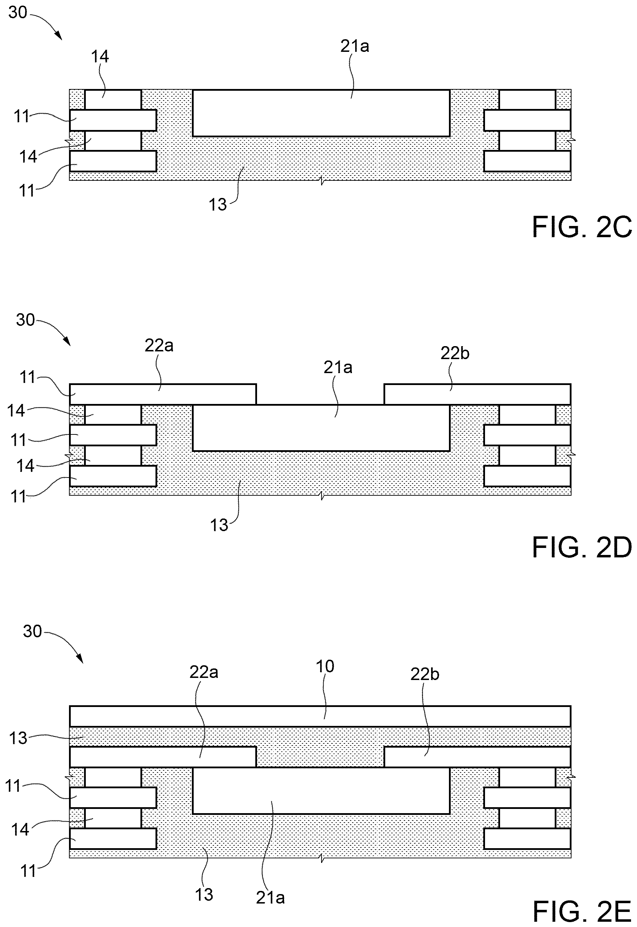

Afterwards, Figure 2c, a conductive layer (corresponding to the next via layer in the stack of the structure of interconnection layers 8), in particular of tungsten material, is deposited, e.g. via sputtering technique, and patterned (via suitable masking) in order to define a metal body 21a of the sensing element 21 within the central cavity 31, and, at a same time, additional conductive vias 14 of the conductive columns 24a, 24b within the lateral recesses 32a, 32b.

As shown in Figure 2d, a metal layer (corresponding to the next additional conductive layer 11 in the stack of the structure of interconnection layers 8), e.g. of aluminium material, is deposited on the wafer 30, and patterned via suitable masking in order to define the first and second electrodes 22a, 22b, contacting the end portions of the sensing element 21 and the conductive columns 24a, 24b (as shown in Figure 2d, a void region remains between the first and second electrodes 22a, 22b, on the sensing element 21).

Then, Figure 2e, standard CMOS processing steps are performed for formation of a further dielectric layer 13 (also filling the above void region) and of the top conductive layer 10.

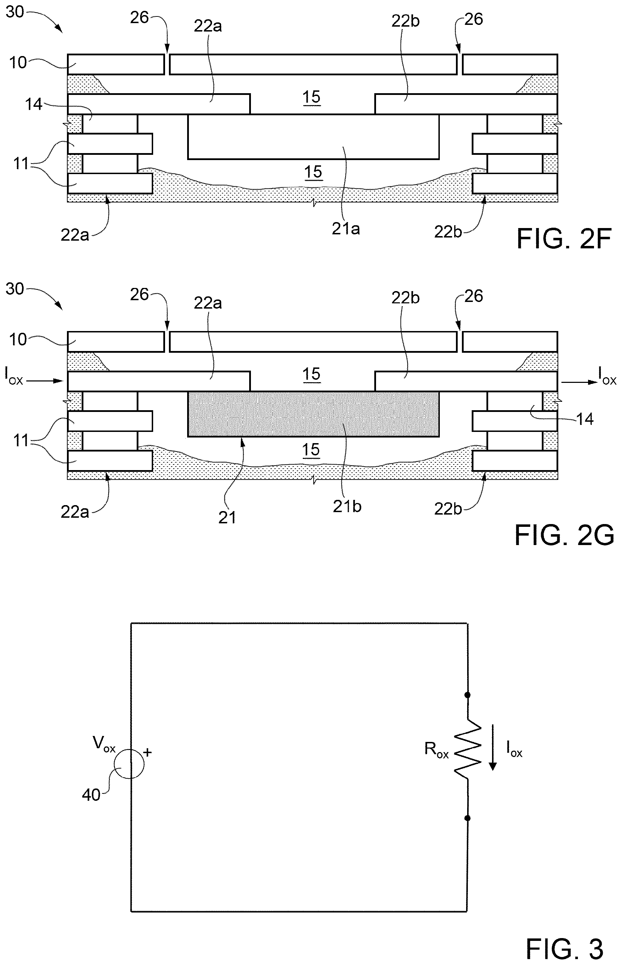

Moreover, as shown in Figure 2f, through holes 26 are then formed through the top conductive layer 10 (only two of these through holes 26 are shown, by way of example).

According to an aspect of the present solution, etching, preferably a wet etching and more preferably a vapour etching via vHF (vapor hydrofluoric acid), is then performed via the through holes 26. In particular, the etching agent penetrates within the structure of interconnection layers 8 and removes the dielectric material of the dielectric layers 13, forming the hollow space 15 around the sensing element 21, which is left suspended above the same hollow space 15, attached to the first and second electrodes 22a, 22b.

A next, subsequent, step of the process envisages, Figure 2g, oxidation of the metal body 21a, in order to define a metal oxide body 21b, and thus complete formation of the sensing element 21 within the structure of interconnection layers 8 of the CMOS wafer 30.

A particular aspect of the present solution envisages performing a so called "self-oxidation" of the metal body 21a; an oxidation current Iₒₓ is made to flow through the metal body 21a via the first and second electrodes 22a, 22b, to cause heating thereof by the Joule effect. This allows to perform oxidation in a standard air atmosphere, possibly avoiding the need of a rich oxygen atmosphere.

According to a possible solution, oxidation is thus performed through "self-heating" of the metal body 21a of the sensing element 21, i.e. without external heater elements (thus simplifying the structure and the manufacturing process).

In this respect, the present Applicant has realized that, if the current (or correspondingly the temperature) of the tungsten wire rises above a certain threshold (which is design dependent, and moreover has also a wide variability given the same design parameters), there is the risk that the wire is broken, separating in two or more parts, and hence it is no longer useful.

Therefore, a right current level (or analogously, a corresponding voltage to cause the desired current level) to heat the metal body 21a of the sensing element 21 by Joule heating has to be determined.

The present Applicant has also realized that a current source may not be suitable for the purpose of causing the above self-oxidation. Indeed, when the wire is oxidized, its resistance increases by a range of 10 to 100 times, so that the Joule heating would be suddenly so high as to cause damage to, or breaking of, the wire.

Accordingly, and as schematically shown in Figure 3, where the metal body 21a of the sensing element 21 is represented as a resistor with oxidation resistance Rₒₓ, an aspect of the present solution envisages the use of a voltage source 40 providing an oxidation voltage Vₒₓ, in order to generate the oxidation current Iₒₓ through the metal body 21a to cause heating by the Joule effect.

In order to determine an optimized value of the oxidation voltage Vₒₓ (not too high, so as not to break the wire, nor too low, so as not to take too long to oxidize), an aspect of the present solution envisages implementation of a suitable oxidation algorithm.

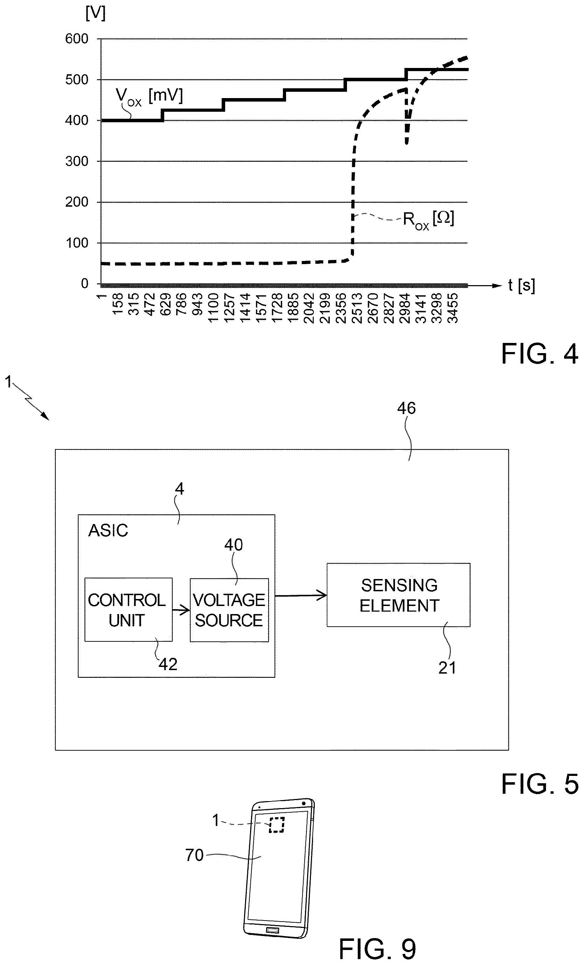

In detail, and with reference also to Figure 4, this algorithm envisages a gradual and step-wise increase of the oxidation voltage Vₒₓ, starting from a value known to be well under a breaking or dangerous limit.

The oxidation voltage Vₒₓ is then increased stepwise, with gradual increases after a preset time interval (during which the voltage value is kept constant); this preset time interval may for example range from 1 to 10 minutes.

At each new value of the oxidation voltage Vₒₓ, the value of the supplied oxidation current Iₒₓ is evaluated, in order to determine the actual value of the oxidation resistance Rₒₓ.

When it is determined that the actual value of the oxidation resistance Rₒₓ has increased by a preset amount with respect to the initial value (e.g. is ten times higher), or has reached a preset threshold, the algorithm envisages to stop heating of the metal body 21a, which is determined to have been sufficiently oxidized (thus completing formation of the sensing element 21).

In particular, heating may be stopped either immediately at the determination of the preset increase of the resistance value or reaching of the threshold, or at the end of the interval in which this determination occurs, or else at the end of a given waiting time starting from this determination.

The above oxidation algorithm may be performed at the end of the CMOS manufacturing process, i.e. with final processing steps executed in the foundry or processing plant, or during testing of the manufactured CMOS wafer 30.

In this case, the above algorithm may be implemented externally from the integrated gas sensor 1, by a suitable control unit monitoring the processing operations. In particular, the oxidation voltage Vₒₓ may be applied via suitably provided connection pads (not shown in the Figures) in the top metal layer 11, and being generated externally to the package (not shown in the Figures) of the same integrated gas sensor 1.

A further possibility envisages again oxidation at the wafer level in the manufacturing plant, in this case using pads that may not be connected to the package. The electrical connection could be done with probe cards or a bed of nails. One advantage of this approach could be the possibility, in certain circumstances, to use an oxygen rich atmosphere, which would lead to a better, faster and more uniform oxidation.

As an alternative, and according to a further aspect of the present solution, self-oxidation can be performed in the field, i.e. during a first operation of the integrated gas sensor 1 (embedded in an electronic device, e.g. a mobile device), by means of an initialization routine that is performed at the first operation of the integrated gas sensor 1. This possibility takes full advantage of the CMOS integration of the sensing element 21 with the related ASIC 4.

In a possible embodiment, the oxidation resistance Rₒₓ could be sensed, and, if determined to be below a threshold, self oxidation could be performed; this solution could be advantageous in avoiding possible incomplete oxidation in case of power interruption just after power up.

In another embodiment, self oxidation may be performed at a start-up phase, or at a first power-on or supplying of electrical energy by an electrical source (e.g. the power supply unit of the mobile device).

In any case, it may be advantageous to store, in a non volatile memory element, information concerning the execution of the self oxidation procedure (in order to avoid further execution of the same procedure).

As schematically shown in Figure 5, ASIC 4 may be provided with a suitable control unit 42, e.g. a microcontroller, a microprocessor, an FPGA (Field Programmable Gate Array) or other processing unit, designed to implement in the field the oxidation algorithm discussed above, when a first operation of the integrated gas sensor 1 is detected.

In this case, ASIC 4 further includes voltage source 40 and suitable feedback element (here not shown) for the control unit 42 to monitor the value of the oxidation current Iₒₓ.

In the same Figure 5, the package of the integrated gas sensor 1 is shown schematically and denoted with 46. In a known manner, package 46 houses the CMOS die 30 and provides suitable electrical connections to the outside, e.g. to an external PCB - Printed Circuit Board, and also allows access to the hollow space 15 internal to the same CMOS die and introduction of the gas or gases to be detected.

Initialization and self-oxidation routine may thus be performed in the field, transparent to the user, by the ASIC 4, allowing to further simplify and streamline the manufacturing process (which in this case does not require the last oxidation step).

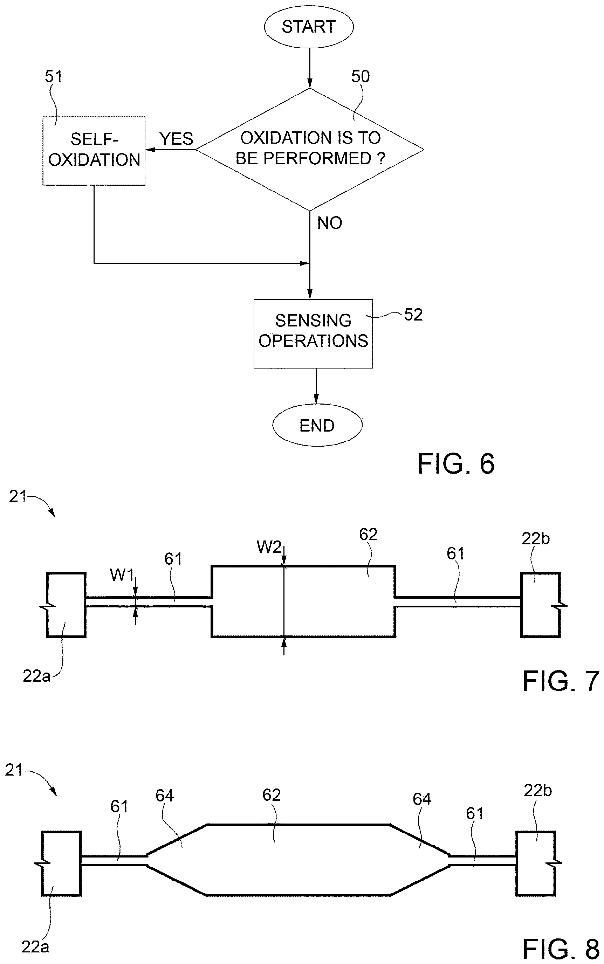

As schematically shown in Figure 6, the initialization and self-oxidation routine may envisage, at a first step 50, determination, by the control unit 42 within the ASIC 4 of the integrated gas sensor 1, that oxidation of the metal body 21a of the sensing element 21 has to be performed.

For example, this determination is made after sensing a start-up or first power-up of the integrated gas sensor 1, or after the resistance of the same metal body 21a is measured and found to be lower than a given threshold.

In case the above determination gives a positive result, self-oxidation of the metal body 21a of the sensing element 21 is performed, at step 51, as previously explained in detail; to this end, control unit 42 may control the voltage source 40 to supply the oxidation voltage Vₒₓ, with gradually increasing steps, while monitoring the value of the oxidation current Iₒₓ.

Self-oxidation ends when a suitable increase of the oxidation resistance Rₒₓ is determined by the same control unit 42; standard gas sensing operations may then be performed, as shown at step 52.

Instead, if at step 50 control unit 40 determines that the self-oxidation has already been performed, standard gas sensing operations are immediately implemented, at step 52.

The present Applicant has further realized that, in order to minimize the noise of the integrated gas sensor 1, it may be desirable to maximize the area oxidized.

However, the present Applicant has realized that with tungsten nano-wires, whatever the length and the shape (e.g. straight line or meander-shape in plan view), usually only a central part reaches the required high temperature (in the order of 800°C - 900°C, ideally) to be fully oxidized. The rest of the wire may be not properly oxidized, and hence may not contribute, or not fully participate, in the gas sensing; this may increase the noise of the gas sensor, which is not desirable.

A further aspect of the present solution thus envisages, as shown in Figure 7, a suitable shape for the sensing element 21, having a wider portion in the centre thereof (Figure 7 refers, by way of example, to a linear overall shape of the sensing element 1, in plan view; however, the same sensing element 21 could have different shapes, such as a meander-shape).

In detail, the sensing element 21 includes, in plan view, lateral sections 61 (connected to the first and second electrodes 22a, 22b) having a first width (transverse to a longitudinal extension thereof) W₁, and a central section 62 having a second width W₂, wherein the second width W₂ is much higher (as a non limitative example, from 10 to 100 times higher) than the first.

In this solution, the lateral portions 61 will be responsible for raising the temperature during Joule heating (or self-heating), both during self-oxidation, and also during the gas sensing operations for setting the operating temperature point. The central section 62 will instead be the one that oxidizes and is then responsible to change its resistance according to the presence of the gases to be detected and the operating temperature set by the lateral sections 61.

Another potential improvement is to avoid changing abruptly from the thin lateral sections 61 to the thick central section 62.

Therefore, a further embodiment, shown in Figure 8, envisages the presence of transition regions 64, to join the lateral sections 61 to the central section 62, having a trapezoidal shape in plan view (therefore having a section increasing from thin to thick, or a tapered shape from the first to the second width W₁, W₂).

This embodiment allows a wider margin of operating temperatures to be set for the oxidation, leading to better yield and reliability.

The advantages of the proposed solution are clear from the foregoing description.

In particular, the integrated gas sensor 1 can be manufactured using mostly standard processing, e.g. CMOS processing, and allows to integrate the sensing element 21 with the related electronics within a single die, e.g. a CMOS die; in particular, an ASIC 4 can be integrated in the substrate 2 of the CMOS die, while the sensing element 21 of the integrated gas sensor 1 can be integrated within the overlying structure of interconnection layers 8.

The discussed solution can be implemented not only with just standard CMOS steps, but with a standard CMOS process, possibly violating some DRC rules to implement the tungsten wire using a standard via layer of a standard CMOS with Al back-end.

The resultant dimensions and the manufacturing costs of the integrated gas sensor 1 are greatly reduced with respect to traditional solutions.

Moreover, the manufacturing process, using mostly standard CMOS processing, is ready for HVM (High Volume Manufacturing) and provide a very short TTM (Time To Market) in the design of the integrated gas sensor 1.

It is also underlined that the discussed processing steps, both in case of execution during the manufacturing process and in the field, advantageously envisage performing oxidation for definition of the sensing element 21, after the formation of the hollow space 15 and etching of the dielectric material in the structure of interconnection layers 8; accordingly, etching of the dielectric material (e.g. silicon oxide) does not affect the (later formed) metal oxide of the sensing element 21.

In other words, the self-oxidation solution allows to leave the oxidation step as a last step of the process, and the metal body 21a of the sensing element 21, e.g. the tungsten wire, may be released before being oxidized. Accordingly, both using wet or dry etching, preferably vHF, the silicon oxide can be easily etched selectively with respect to the metal material, e.g. tungsten. On the contrary, if the sensing element were to be released after oxidation (it is underlined that this release has to be done towards the end of the process, in order to avoid problems in dealing with the hollow space during subsequent processing steps), it would be very difficult to selectively etch the metal oxide with respect to the silicon oxide, that would in this case surround the sensing element 21.

The features discussed above are particularly advantageous, in case the integrated gas sensor 1 is embedded inside a mobile phone, a tablet, or in general a mobile device or handset; indeed, the discussed integrated gas sensor 1 allows to achieve the low cost, low power and low size requirements that are generally required to mobile devices.

Therefore, the discussed integrated gas sensor 1 enables a mobile handset having gas or multi-gas sensing capability, as shown schematically in Figure 9, showing a mobile device, generally denoted with 70, being provided with the integrated gas sensor 1.

This mobile handset 70, with the right software or applications (the so called "Apps") may be configured to implement a breathalyzer, to monitor the air quality, to detect gas leakages, to sense the quality of food and beverages, and/or to detect illness or malaise, e.g. smelling the breath of the user while he/she speaks. In the later case, the mobile handset, with the right software or App, could offer, for example, the possibility to buy on-line, or to otherwise acquire, a medicine or other solution to improve the health of the user.

Finally, it is clear that modifications and variations may be made to what has been described and illustrated herein, without thereby departing from the scope of the present invention, as defined in the annexed claims.

In particular, modifications may be envisaged in the manufacturing steps for the formation of the sensing element 21 within the CMOS structure.

For example, the top conductive layer 10 could be used for the formation of the first and second electrodes 22a, 22b and the suspension attachment of the sensing element 21; in this case, the sensing element 21 would be fully exposed, so the package should provide sufficient mechanical protection from the external environment.

Analogously, the sensing element 21 could be manufactured at the level of a different conductive layers 11 in the structure of interconnection layers 8.

Moreover, more than two electrodes could be coupled to the sensing element 21, in order to implement a so called "4-wire measuring scheme", i.e. using separate pairs of current-carrying and voltage-sensing electrodes to improve the sensing performances, at least in certain operating conditions.

It is also underlined that the discussed solution may be implemented in a Bi-CMOS process, or in general in any other standard semiconductor microelectronic process for manufacturing solid state electronics integrated circuits, preferably provided with tungsten vias (usually found in Al back-end processing).

The solution envisaging the initialization and self-oxidation in the field, as well as self-heating, could be used also in case other manufacturing processes were used to manufacture the sensing element 21 (even different from the CMOS processing discussed above).

The sensing element 21 would in any case be coupled to a corresponding ASIC 4 (manufactured in a same or distinct die, and possibly provided within a same package), including a suitable control unit 42 to monitor and control the oxidation process, autonomously and independently from external equipments. Indeed, this solution would in any case allows an improvement and simplification of the related manufacturing process, particularly with respect to the release of the sensing element (or etching of the silicon oxide surrounding it).

Furthermore, other algorithms could be considered for implementing the self-oxidation in the sensing element 21, with the general aim to minimize the time required for the oxidation, while achieving an optimized oxidation.

## Claims

1. An integrated gas sensor (1) having a gas sensing element (21), comprising:
a substrate (2) including semiconductor material; and
a structure of interconnection layers (8), arranged above the substrate (2) and including a number of stacked conductive layers (9, 10, 11) and dielectric layers (13), wherein the gas sensing element (21) is integrated within the structure of interconnection layers (8), and at least one electrode (22a, 22b) is provided within the structure of interconnection layers (8), electrically connected to the gas sensing element (21) and designed to provide an electric current (Iₒₓ) to the gas sensing element (21) in order to cause heating thereof;
**characterized by** further including an integrated electronic circuit (4) within the substrate (2), operatively coupled to the gas sensing element (21) and configured to cause the electric current (Iₒₓ) to flow through the gas sensing element (21), in at least one operating condition; the integrated electronic circuit (4) including a voltage source (40) and a control unit (42), configured to control the voltage source (40) in order to provide the electric current (Iₒₓ) to the gas sensing element (21) during the at least one operating condition, thereby causing oxidation of a metal body (21a) of the gas sensing element (21).

2. The sensor according to claim 1, wherein the gas sensing element (21) includes a metal body (21a), made of tungsten.

3. The sensor according to claim 1 or 2, wherein a hollow space (15) is provided within the structure of interconnection layers (8), the gas sensing element (21) being arranged within the hollow space (15).

4. The sensor according to claim 3, wherein the gas sensing element (21) is attached to a supporting conductive layer (11) among the stacked conductive layers (9, 10, 11), the supporting conductive layer (11) also defining the at least one electrode (22a, 22b); wherein at least one conductive columns (24a, 24b) is provided within the structure of interconnection layers (8), electrically connected to the at least one electrode (22a, 22b) and extending from the same electrode (22a, 22b) towards the substrate (2); the at least one conductive columns (24a, 24b) including a stack-up of portions of conductive layers (11), and conductive vias (14) defined through respective dielectric layers (13).

5. The sensor according to claim 4, wherein the gas sensing element (21) is realized in a same layer as at least one of the conductive vias (14).

6. The sensor according to any of the preceding claims, wherein the gas sensing element (21) has a longitudinal extension, and includes lateral portions (61) with a first dimension (W₁) transverse to the longitudinal extension, and a central portion (62) with a corresponding second dimension (W₂) transverse to the longitudinal extension; the second dimension (W₂) being larger than the first dimension (W₁); wherein at least one of the lateral portions (61) is connected to the at least one electrode (22a, 22b).

7. The sensor according to claim 6, wherein the gas sensing element (21) further includes transition portions (64), connecting the central portion (62) to the lateral portions (61) and having a tapered shape from the first (W₁) to the second (W₂) dimension.

8. The sensor according to any of the preceding claims, wherein the control unit (42) is configured to determine the occurrence of the operating condition, and, in response thereto, cause oxidation of the metal body (21a), by application of a gradually increasing oxidation voltage (Vₒₓ), until a resistance value (Rₒₓ) of the metal body (21a) reaches an oxidation threshold.

9. The sensor according to any of the preceding claims, wherein the operating condition corresponds to at least one of: a start-up or a first power-up of the integrated gas sensor (1); a resistance value (Rₒₓ) of the metal body (21a) being lower than a preset threshold.

10. A mobile device (70) including the integrated gas sensor (1) according to any of the preceding claims.

11. The device according to claim 10, having at least one of the following functions: breathalyzer; air quality monitoring; food and/or beverage inspection; gas leakage detection; breath analyzer for illness detection.

12. A process for manufacturing an integrated gas sensor (1) having a gas sensing element (21), comprising:
providing a substrate (2) including a semiconductor material;
forming a structure of interconnection layers (8) above the substrate (2), including a number of stacked conductive layers (9, 10, 11) and dielectric layers (13);
integrating the gas sensing element (21) and at least one electrode (22a, 22b), electrically connected to the gas sensing element (21), within the structure of interconnection layers (8), the at least one electrode (22a, 22b) designed to provide an electric current (Iₒₓ) to the gas sensing element (21) in order to cause heating thereof;
**characterized in that** integrating the gas sensing element (21) includes:
forming a hollow space (15) within the structure of interconnection layers (8) using a wet or vHF etching step to remove a portion of the dielectric layers (13); and
after formation of the hollow space (15), causing oxidation of a metal body (21a) of the gas sensing element (21), by application of the electric current (Iₒₓ) to the at least one electrode (22a, 22b).

13. The process according to claim 12, wherein integrating the gas sensing element (21) includes:
selectively removing a portion of at least one dielectric layer (13), in order to define a cavity (31);
forming a metal layer on the at least one dielectric layer (13), in order to fill the cavity (31) with a metal body (21a) of the gas sensing element (21);
forming a supporting conductive layer (11), among the stacked conductive layers (9, 10, 11), on the metal layer, and defining the same supporting conductive layer (11) in order to provide the at least one electrode (22a, 22b), connected to the metal body (21a);
etching the dielectric layer (13) around the metal body (21a), via a wet or vHF etching, in order to define a hollow space (15) within the structure of interconnection layers (8); wherein the gas sensing element (21) is arranged within the hollow space (15) and the gas sensing element (21) is attached to the supporting conductive layer (11).

14. The process according to any of claims 12-13, wherein providing the substrate (2) includes forming an integrated electronic circuit (4) within the substrate (2), operatively coupled to the gas sensing element (21) and configured to cause the electric current (Iₒₓ) to flow through the gas sensing element (21).

15. A method for operating an integrated gas sensor (1), having a gas sensing element (21) and comprising:
a substrate (2) including semiconductor material; and
a structure of interconnection layers (8), arranged above the substrate (2) and including a number of stacked conductive layers (9, 10, 11) and dielectric layers (13); wherein the gas sensing element (21) is integrated within the structure of interconnection layers (8), and at least one electrode (22a, 22b) is provided within the structure of interconnection layers (8), electrically connected to the gas sensing element (21) and designed to provide an electric current (Iₒₓ) to the gas sensing element (21) in order to cause heating thereof,
**characterized by**:
at a first operation of the integrated gas sensor (1), causing oxidation of the gas sensing element (21) of the gas integrated sensor (1), by application of the electric current (Iₒₓ) to the at least one electrode (22a, 22b).

16. The method according to claim 15, being executed in the field, after manufacturing of the integrated gas sensor (1).

## Patentansprüche

1. Integrierter Gassensor (1) mit einem Gaserfassungselement (21), umfassend:
ein Substrat (2), das Halbleitermaterial enthält; und
eine Struktur von Verbindungsschichten (8), die über dem Substrat (2) angeordnet ist und eine Anzahl von gestapelten leitfähigen Schichten (9, 10, 11) und dielektrischen Schichten (13) enthält,
wobei das Gaserfassungselement (21) innerhalb der Struktur von Verbindungsschichten (8) integriert ist und zumindest eine Elektrode (22a, 22b) innerhalb der Struktur von Verbindungsschichten (8) bereitgestellt ist, und zwar elektrisch verbunden mit dem Gaserfassungselement (21) und ausgelegt, einen elektrischen Strom (Iₒₓ) an das Gaserfassungselement (21) bereitzustellen, um dessen Erwärmung zu bewirken,
**dadurch gekennzeichnet, dass** er ferner eine integrierte elektronische Schaltung (4) innerhalb des Substrats (2) enthält, die operativ mit dem Gaserfassungselement (21) gekoppelt ist und konfiguriert ist, den elektrischen Strom (I-ₒₓ) zu veranlassen, durch das Gaserfassungselement (21) zu strömen, und zwar in zumindest einem Betriebszustand; wobei die integrierte elektronische Schaltung (4) eine Spannungsquelle (40) und eine Steuer- bzw. Regeleinheit (42) enthält, die konfiguriert sind, die Spannungsquelle (40) zu steuern bzw. zu regeln, um dem Gaserfassungselement (21) während des zumindest einen Betriebszustands den elektrischen Strom (Iₒₓ) bereitzustellen, wodurch eine Oxidation eines Metallkörpers (21a) des Gaserfassungselements (21) bewirkt wird.

2. Sensor nach Anspruch 1, wobei das Gaserfassungselement (21) einen Metallkörper (21a) enthält, der aus Wolfram besteht.

3. Sensor nach Anspruch 1 oder 2, wobei ein Hohlraum (15) innerhalb der Struktur der Verbindungsschichten (8) bereitgestellt ist, wobei das Gaserfassungselement (21) innerhalb des Hohlraumes (15) angeordnet ist.

4. Sensor nach Anspruch 3, wobei das Gaserfassungselement (21) an einer tragenden bzw. stützenden leitfähigen Schicht (11) der gestapelten leitfähigen Schichten (9, 10, 11) angebracht ist, wobei die tragende leitfähige Schicht (11) auch die zumindest eine Elektrode (22a, 22b) definiert; wobei zumindest eine leitfähige Säule (24a, 24b) innerhalb der Struktur der Verbindungsschichten (8) bereitgestellt ist (2), elektrisch mit der zumindest einen Elektrode (22a, 22b) verbunden ist und sich von derselben Elektrode (22a, 22b) zu dem Substrat (2) erstreckt; wobei die zumindest eine leitfähige Säule (24a, 24b) einen Stapel von Abschnitten von leitfähigen Schichten (11) und leifähigen Vias (14) enthält, die durch jeweilige dielektrische Schichten (13) definiert sind.

5. Sensor nach Anspruch 4, wobei das Gaserfassungselement (21) in einer gleichen Schicht realisiert ist wie zumindest eines der leitenden Vias (14).

6. Sensor nach einem der vorhergehenden Ansprüchen, wobei das Gaserfassungselement (21) eine Längserstreckung aufweist und laterale Abschnitte (61) mit einer ersten Abmessung (W₁) quer zu der Längserstreckung und einen zentralen Abschnitt (62) mit einer entsprechenden zweiten Abmessung (W₂) quer zu der Längserstreckung enthält; wobei die zweite Abmessung (W₂) größer ist als die erste Abmessung (W₁); wobei zumindest einer der lateralen Abschnitte (61) mit der zumindest einen Elektrode (22a, 22b) verbunden ist.

7. Sensor nach Anspruch 6, wobei das Gaserfassungselement (21) ferner Übergangsabschnitte (64) enthält, die den zentralen Abschnitt (62) mit den lateralen Abschnitten (61) verbinden und eine sich verjüngende Form von der ersten (W₁) zu der zweiten Abmessung (W₂) aufweisen.

8. Sensor nach einem der vorhergehenden Ansprüche, wobei die Steuer- bzw. Regeleinheit (42) konfiguriert ist, das Auftreten des Betriebszustands zu bestimmen und ansprechend darauf eine Oxidation des Metallkörpers (21a) zu bewirken, und zwar durch Anlegen einer allmählich ansteigenden Oxidationsspannung (Vₒₓ), bis ein Widerstandswert (Rₒₓ) des Metallkörpers (21a) einen Oxidationsschwelle erreicht.

9. Sensor nach einem der vorhergehenden Ansprüche, wobei der Betriebszustand zumindest einem von entspricht: einer Inbetriebnahme oder einem ersten Einschalten des integrierten Gassensors (1); wobei der Widerstandswert (Rₒₓ) des Metallkörpers (21a) niedriger ist als ein festgelegter Schwellenwert.

10. Mobile Vorrichtung (70), enthaltend den integrierten Gassensor (1) nach einem der vorhergehenden Ansprüche.

11. Vorrichtung nach Anspruch 10, mit zumindest einer der folgenden Funktionen:
Atemalkoholbestimmung; Überwachung der Luftqualität; Lebensmittel- und/oder Getränkeinspektion; Gasleckdetektion; Atemanalysator zur Krankheitserkennung.

12. Verfahren zum Herstellen eines integrierten Gassensors (1), der ein Gaserfassungselement (21) aufweist, umfassend:
Bereitstellen eines Substrats (2), das ein Halbleitermaterial enthält;
Bilden einer Struktur von Verbindungsschichten (8) über dem Substrat (2), die eine Anzahl von gestapelten leitfähigen Schichten (9, 10, 11) und dielektrischen Schichten (13) enthält,
Integrieren des Gaserfassungselements (21) und zumindest einer Elektrode (22a, 22b), elektrisch verbunden mit dem Gaserfassungselement (21), innerhalb die Struktur von Verbindungsschichten (8), wobei die zumindest eine Elektrode (22a, 22b) ausgelegt ist, einen elektrischen Strom (Iₒₓ) an das Gaserfassungselement (21) bereitzustellen, um dessen Erwärmung zu bewirken,
**dadurch gekennzeichnet, dass** das Integrieren des Gaserfassungselements (21) ferner umfasst:
Bilden eines Hohlraums (15) innerhalb der Struktur von Verbindungsschichten (8) unter Verwendung eines Nass- oder vHF-Ätzschritts, um einen Abschnitt der dielektrischen Schichten (13) zu entfernen; und
nach dem Bilden des Hohlraums (15), Bewirken einer Oxidation eines Metallkörpers (21a) des Gaserfassungselements (21) durch Anlegen des elektrischen Stroms (Iₒₓ) an die zumindest eine Elektrode (22a, 22b).

13. Verfahren nach Anspruch 12, wobei das Integrieren des Gaserfassungselements (21) umfasst:
selektives Entfernen eines Abschnitts zumindest einer dielektrischen Schicht (13), um eine Kavität (31) zu definieren;
Bilden einer Metallschicht auf der zumindest einen dielektrischen Schicht (13), um die Kavität (31) mit einem Metallkörper (21a) des Gaserfassungselements (21) zu füllen;
Bilden einer stützenden bzw. tragenden leitfähigen Schicht (11) der gestapelten leitfähigen Schichten (9, 10, 11) auf der Metallschicht und Definieren derselben tragenden leitfähigen Schicht (11), um die zumindest eine Elektrode (22a, 22b bereitzustellen, verbunden mit dem Metallkörper (21a);
Ätzen der dielektrischen Schicht (13) um den Metallkörper (21a) herum über ein Nass- oder vHF-Ätzen, um einen Hohlraum (15) innerhalb der Struktur von Verbindungsschichten (8) zu definieren; wobei das Gaserfassungselement (21) innerhalb des Hohlraums (15) angeordnet ist und das Gaserfassungselement (21) an der tragenden leitfähigen Schicht (11) angebracht ist.

14. Verfahren nach einem der Ansprüche 12-13, wobei das Bereitstellen des Substrats (2) das Bilden einer integrierten elektronischen Schaltung (4) innerhalb des Substrats (2) umfasst, die operativ mit dem Gaserfassungselement (21) gekoppelt ist und konfiguriert ist, den elektrischen Strom (Iₒₓ) zu veranlassen, durch das Gaserfassungselement (21) fließen.

15. Verfahren zum Betreiben eines integrierten Gassensors (1), der ein Gaserfassungselement (21) aufweist und umfasst:
ein Substrat (2), das Halbleitermaterial enthält; und
eine Struktur von Verbindungsschichten (8), die über dem Substrat (2) angeordnet ist und eine Anzahl von gestapelten leitfähigen Schichten (9, 10, 11) und dielektrischen Schichten (13) enthält,
wobei das Gaserfassungselement (21) innerhalb der Struktur von Verbindungsschichten (8) integriert ist und zumindest eine Elektrode (22a, 22b) innerhalb der Struktur von Verbindungsschichten (8) bereitgestellt ist, und zwar elektrisch verbunden mit dem Gaserfassungselement (21) und ausgelegt, einen elektrischen Strom (Iₒₓ) an das Gaserfassungselement (21) bereitzustellen, um dessen Erwärmung zu bewirken,
**gekennzeichnet durch**:
bei einem ersten Betrieb des integrierten Gassensors (1), Bewirken einer Oxidation des Gaserfassungselements (21) des integrierten Gassensors (1) durch Anlegen des elektrischen Stroms (Iₒₓ) an die zumindest einer Elektrode (22a , 22b).

16. Verfahren nach Anspruch 15, ausgeführt auf dem Gebiet bzw. im Feld, nach Herstellung des integrierten Gassensors (1).

## Revendications

1. Capteur de gaz intégré (1) ayant un élément de détection de gaz (21), comprenant :
un substrat (2) comportant un matériau semi-conducteur ; et
une structure de couches d'interconnexion (8), agencée au-dessus du substrat (2) et comportant un nombre de couches conductrices empilées (9, 10, 11) et de couches diélectriques (13), dans lequel l'élément de détection de gaz (21) est intégré au sein de la structure de couches d'interconnexion (8), et au moins une électrode (22a, 22b) est prévue au sein de la structure de couches d'interconnexion (8), raccordée électriquement à l'élément de détection de gaz (21) et conçue pour fournir un courant électrique (Iₒₓ) à l'élément de détection de gaz (21) afin de l'amener à chauffer ;
**caractérisé en ce qu'**il comporte en outre un circuit électronique intégré (4) au sein du substrat (2), couplé opérationnellement à l'élément de détection de gaz (21) et configuré pour amener le courant électrique (Iₒₓ) à circuler à travers l'élément de détection de gaz (21), dans au moins une condition de fonctionnement ; le circuit électronique intégré (4) comportant une source de tension (40) et une unité de commande (42), configurée pour commander la source de tension (40) afin de fournir le courant électrique (Iₒₓ) à l'élément de détection de gaz (21) pendant l'au moins une condition de fonctionnement, provoquant ainsi une oxydation d'un corps en métal (21a) de l'élément de détection de gaz (21).

2. Capteur selon la revendication 1, dans lequel l'élément de détection de gaz (21) comporte un corps en métal (21a), réalisé en tungstène.

3. Capteur selon la revendication 1 ou 2, dans lequel un espace creux (15) est prévu au sein de la structure de couches d'interconnexion (8), l'élément de détection de gaz (21) étant agencé au sein de l'espace creux (15).

4. Capteur selon la revendication 3, dans lequel l'élément de détection de gaz (21) est fixé à une couche conductrice de support (11) parmi les couches conductrices empilées (9, 10, 11), la couche conductrice de support (11) définissant également l'au moins une électrode (22a, 22b) ; dans lequel au moins une colonne conductrice (24a, 24b) est prévue au sein de la structure de couches d'interconnexion (8), raccordée électriquement à l'au moins une électrode (22a, 22b) et partant de cette même électrode (22a, 22b) vers le substrat (2) ; l'au moins une colonne conductrice (24a, 24b) comportant un empilement de portions de couches conductrices (11), et des trous de liaison conducteurs (14) définis à travers des couches diélectriques (13) respectives.

5. Capteur selon la revendication 4, dans lequel l'élément de détection de gaz (21) est réalisé dans une même couche qu'au moins l'un des trous de liaison conducteurs (14).

6. Capteur selon l'une quelconque des revendications précédentes, dans lequel l'élément de détection de gaz (21) a une extension longitudinale, et comporte des portions latérales (61) d'une première dimension (W₁) transversale à l'extension longitudinale, et une portion centrale (62) d'une seconde dimension (W₂) correspondante transversale à l'extension longitudinale ; la seconde dimension (W₂) étant plus grande que la première dimension (W₁) ; dans lequel au moins l'une des portions latérales (61) est raccordée à l'au moins une électrode (22a, 22b).

7. Capteur selon la revendication 6, dans lequel l'élément de détection de gaz (21) comporte en outre des portions de transition (64), raccordant la portion centrale (62) aux portions latérales (61) et ayant une forme effilée de la première (W₁) à la seconde (W₂) dimension.

8. Capteur selon l'une quelconque des revendications précédentes, dans lequel l'unité de commande (42) est configurée pour déterminer la survenue de la condition de fonctionnement, et, en réponse à celle-ci, provoquer une oxydation du corps en métal (21a), par l'application d'une tension d'oxydation (Vₒₓ) progressivement croissante, jusqu'à ce qu'une valeur de résistance (Rₒₓ) du corps en métal (21a) atteigne un seuil d'oxydation.

9. Capteur selon l'une quelconque des revendications précédentes, dans lequel la condition de fonctionnement correspond à au moins l'un parmi : un démarrage ou une première mise sous tension du capteur de gaz intégré (1) ; une valeur de résistance (Rₒₓ) du corps en métal (21a) étant inférieure à un seuil préétabli.

10. Dispositif mobile (70) comportant le capteur de gaz intégré (1) selon l'une quelconque des revendications précédentes.

11. Dispositif selon la revendication 10, ayant au moins l'une des fonctions suivantes : éthylomètre ; surveillance de la qualité de l'air ; vérification des aliments et/ou des boissons ; détection de fuite de gaz ; analyseur de gaz respiratoire pour une détection de maladie.

12. Procédé de fabrication d'un capteur de gaz intégré (1) ayant un élément de détection de gaz (21), comprenant :
la fourniture d'un substrat (2) comportant un matériau semi-conducteur ;
la formation d'une structure de couches d'interconnexion (8) au-dessus du substrat (2), comportant un nombre de couches conductrices empilées (9, 10, 11) et de couches diélectriques (13) ;
l'intégration de l'élément de détection de gaz (21) et d'au moins une électrode (22a, 22b), raccordée électriquement à l'élément de détection de gaz (21), au sein de la structure de couches d'interconnexion (8), l'au moins une électrode (22a, 22b) étant conçue pour fournir un courant électrique (Iₒₓ) à l'élément de détection de gaz (21) afin de l'amener à chauffer ;
**caractérisé en ce que** l'intégration de l'élément de détection de gaz (21) comporte :
la formation d'un espace creux (15) au sein de la structure de couches d'interconnexion (8) à l'aide d'une étape de gravure humide ou vHF pour éliminer une portion des couches diélectriques (13) ; et
après formation de l'espace creux (15), le fait de provoquer l'oxydation d'un corps en métal (21a) de l'élément de détection de gaz (21), par l'application du courant électrique (Iₒₓ) à l'au moins une électrode (22a, 22b).

13. Procédé selon la revendication 12, dans lequel l'intégration de l'élément de détection de gaz (21) comporte :
l'élimination sélective d'une portion d'au moins une couche diélectrique (13), afin de définir une cavité (31) ;
la formation d'une couche en métal sur l'au moins une couche diélectrique (13), afin de remplir la cavité (31) avec un corps en métal (21a) de l'élément de détection de gaz (21) ;
la formation d'une couche conductrice de support (11), parmi les couches conductrices empilées (9, 10, 11), sur la couche en métal, et la définition de cette même couche conductrice de support (11) afin de fournir l'au moins une électrode (22a, 22b), raccordée au corps en métal (21a) ;
la gravure de la couche diélectrique (13) autour du corps en métal (21a), via une gravure humide ou vHF, afin de définir un espace creux (15) au sein de la structure de couches d'interconnexion (8) ; dans lequel l'élément de détection de gaz (21) est agencé au sein de l'espace creux (15) et l'élément de détection de gaz (21) est fixé à la couche conductrice de support (11).

14. Procédé selon l'une quelconque des revendications 12 et 13, dans lequel la fourniture du substrat (2) comporte la formation d'un circuit électronique intégré (4) au sein du substrat (2), couplé opérationnellement à l'élément de détection de gaz (21) et configuré pour amener le courant électrique (Iₒₓ) à circuler à travers l'élément de détection de gaz (21).

15. Méthode de fonctionnement d'un capteur de gaz intégré (1), ayant un élément de détection de gaz (21) et comprenant :
un substrat (2) comportant un matériau semi-conducteur ; et
une structure de couches d'interconnexion (8), agencée au-dessus du substrat (2) et comportant un nombre de couches conductrices empilées (9, 10, 11) et de couches diélectriques (13) ; dans laquelle l'élément de détection de gaz (21) est intégré au sein de la structure de couches d'interconnexion (8), et au moins une électrode (22a, 22b) est prévue au sein de la structure de couches d'interconnexion (8), raccordée électriquement à l'élément de détection de gaz (21) et conçue pour fournir un courant électrique (Iₒₓ) à l'élément de détection de gaz (21) afin de l'amener à chauffer,
**caractérisée par** :
lors d'un premier fonctionnement du capteur de gaz intégré (1), le fait de provoquer l'oxydation de l'élément de détection de gaz (21) du capteur de gaz intégré (1), par l'application du courant électrique (Iₒₓ) à l'au moins une électrode (22a, 22b).

16. Méthode selon la revendication 15, exécutée sur le terrain, après fabrication du capteur de gaz intégré (1).
